# EUROPEAN PATENT APPLICATION

(11) **EP 3 279 338 A1**
(43) Date of publication of application: **07.02.2018**
(21) Application number: 16772779.1
(22) Date of filing: 29.03.2016
(51) Int. Cl.: C12Q 1/68, C12N 15/09, G01N 21/64

(54) **GENE MUTATION DETECTION METHOD AND FLUORESCENCE-LABELED OLIGONUCLEOTIDE USED IN SAME**

(30) Priority: 31.03.2015 JP 2015070642
(71) Applicant: Nippon Steel & Sumikin Eco-Tech Corporation, Tokyo 104-0031 (JP)
(72) Inventor: YAMAGUCHI, Masahiro, Tokyo 104-0031 (JP); KURATA, Shinya, Tokyo 104-0031 (JP); KOMATSU, Norio, Tokyo 113-8421 (JP); MORISHITA, Soji, Tokyo 113-8421 (JP)
(74) Representative: J A Kemp
(86) International application number: PCT/JP2016/060011
(87) International publication number: WO 2016/158898

(57) **Abstract**

Provided are techniques by which a mutant gene in a gene group comprising a large number of wild-type genes can be detected with high sensitivity and in a rapid and simple way. Provided is a method for measuring a nucleic acid for the purpose of specifically detecting a genotype as an examination target from subjects which are genes or specimens likely to have a plurality of genetic polymorphisms, wherein the measurement method comprises hybridizing a fluorescent dye-labeled oligonucleotide with a genotype other than the examination target to suppress the gene amplification of such genotype while at the same time, hybridizing the same fluorescence-labeled oligo as described above with an amplification product derived from the genotype of the examination target amplified in the same gene amplification step as described above, and specifically detecting the genotype of the examination target based on a change in the fluorescence intensity of the fluorescent dye before and after the hybridization. A fluorescence-labeled oligo that can be used in the above described method is also provided.

SEQUENCE LISTING

<110>  NIPPON STEEL & SUMIKIN Eco-Tec Corporation
       JUNTENDO UNIVERSITY

<120>  A method of detecting mutated gene and fluorescent-labeled
       oligonucleotides  used therein

<130>  FP-206PCT

<150>  JP 2015-70642
<151>  2015-03-31

<160>  8

<170>  PatentIn version 3.5

<210>  1
<211>  29
<212>  DNA
<213>  Artificial Sequence

<220>
<223>  Primer

<400>  1
atctatagtc atgctgaaag taggagaaa                                         29


<210>  2
<211>  30
<212>  DNA
<213>  Artificial Sequence

<220>
<223>  Primer

<400>  2
ctgaatagtc ctacagtgtt ttcagtttca                                        30


<210>  3
<211>  16
<212>  DNA
<213>  Artificial Sequence

<220>
<223>  Probe

<400>  3
cacagacaca tactcc                                                       16


<210>  4
<211>  20
<212>  DNA
<213>  Artificial Sequence

<220>
<223>  Primer

<400>  4
cacctagctg tgatcctgaa                                                   20

<210>  5
<211>  16
<212>  DNA
<213>  Artificial Sequence

<220>
<223>  Probe

<400>  5
cacagacaca tactcc                                                        16


<210>  6
<211>  21
<212>  DNA
<213>  Artificial Sequence

<220>
<223>  Primer

<400>  6
tgaccgctct gcatctagtg c                                                  21


<210>  7
<211>  20
<212>  DNA
<213>  Artificial Sequence

<220>
<223>  Primer

<400>  7
ggtcacagag cgaaccaaga                                                    20


<210>  8
<211>  17
<212>  DNA
<213>  Artificial Sequence

<220>
<223>  Probe

<400>  8
actgccacct cagcagc                                                       17

## Description

### Technical Field

The present invention relates to an oligonucleotide, a method for specifically amplifying a nucleic acid using the oligonucleotide, a method for measuring a nucleic acid using the oligonucleotide, and a method for analyzing data obtained by the aforementioned methods. Specifically, the present invention relates to: a specific amplification method, which comprises performing a nucleic acid amplification reaction with an oligonucleotide remaining hybridized with a target nucleic acid, so as to suppress the amplification of the target nucleic acid; various types of methods for measuring various types of nucleic acids using a fluorescence-labeled oligo, wherein the measurement methods are based on a principle that involves measuring how much of light emission from a fluorescent dye that occurs when the fluorescence-labeled oligo is hybridized with a target nucleic acid decreases before and after hybridization; and an oligonucleotide used in those methods.

### Background Art

Mutation of a gene is a cause of cancer. Accordingly, as a method for discovering cancer at an early stage, a gene mutation test that applies a gene amplification method such as PCR is considered important.

Such a gene mutation test is important not only for early discovery of cancer, but also for determination of therapeutic tactics. One example is the evaluation of the beneficial effects of Gefitinib (tyrosine kinase inhibitor) for lung cancer patients having an EGFR (epidermal growth factor receptor) gene mutation. Among such EGFR gene mutations, Gefitinib is known to have high success rate in cases involving a deletion mutation of exon 19 and a leucine → arginine mutation of exon 21 codon 858.

However, in general, a majority of cells contained in a specimen are normal cells, and only a few cells contain mutant genes. Accordingly, a genotype determination method that uses a common gene amplification technique has only low detection sensitivity due to the presence of a large number of wild-type genes, and this has been a problem for use as a clinical test.

Moreover, with regard to a JAK2 gene mutation (JAK2V617F) in myeloproliferative neoplasm, measurement of a quantitative change in its allele frequency is of high clinical significance, and detection of mutant genes existing at a very small frequency in wild-type genes is extremely important for determining an involvement at an early stage.

As described in the above example, detection of mutant genes with high sensitivity in a gene group comprising a large number of wild-type genes is of extreme clinical importance and it is desired to develop a method capable of realizing such high sensitivity detection.

Nagai et al. have attempted to detect, with high sensitivity, 11 types of mutations generated in the EGFR gene according to a method called "PNA-LNA PCR clamp." As a result, it was possible to detect a mutation with an allele frequency of 0.1% (Nagai et. al., Cancer Research, 65: 7276-7282, 2005.: Non Patent Literature 1). However, in this method, it is necessary to use three types of oligonucleotides consisting of two fluorescence-labeled oligonucleotides, one for detection of mutations and the other for confirmation of amplification, and a clamp primer, and application to other gene mutations is considered to involve cumbersome designs. In addition, this existing method has the need to use many oligonucleotides, which is recognized to be a problem that should be solved before it can be applied in clinical tests which need cost reduction.

Miyano et al. have attempted to detect a KRAS gene mutation according to a PCR clamp method using PNA (Miyano et. al., Experimental And Therapeutic Medicine, 4: 790-794, 2012.: Non Patent Literature 2). In this method, an amplification reaction is carried out using a single clamp primer. However, since detection is carried out using SYBR Green, it is impossible to accurately determine whether or not signals are derived from the mutant genes, and thus, it is considered difficult to apply this method to clinical sites which required great accuracy.

### Citation List

### Non Patent Literature

Non Patent Literature 1: Nagai et. al., Cancer Research, 65: 7276-7282, 2005.
Non Patent Literature 2: Miyano et. al., Experimental And Therapeutic Medicine, 4: 790-794, 2012.

### Summary of Invention

### Technical Problem

It is an object of the present invention to provide a method for specifically amplifying a nucleic acid and a method for measuring a nucleic acid, both methods using a fluorescence-labeled oligo and serving as techniques by which a mutant gene in a gene group comprising a large number of wild-type genes can be detected with high sensitivity and in a rapid and simple way.

### Solution to Problem

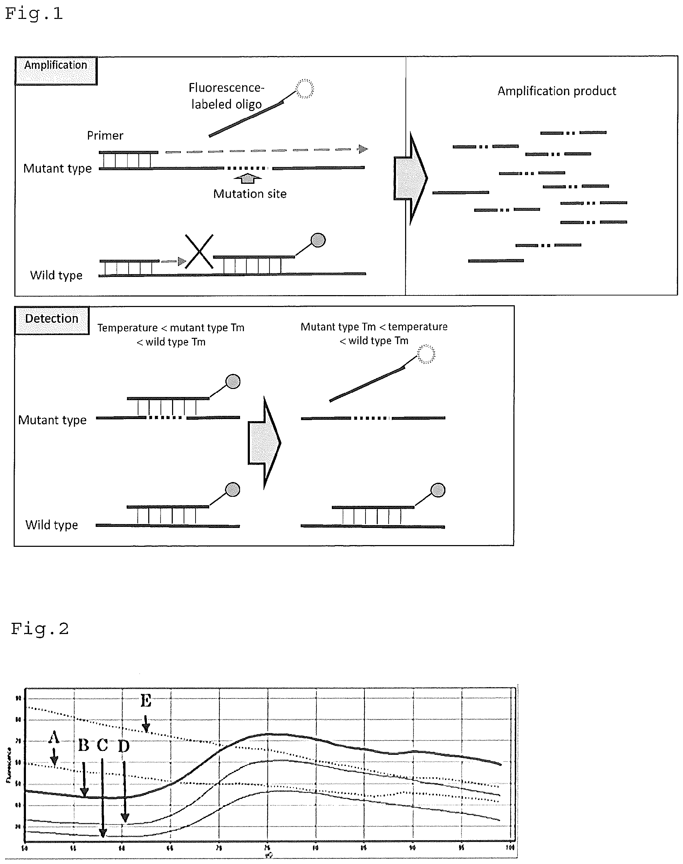

The present inventors have conducted intensive studies on a method for specifically amplifying a mutant gene and a method that follows to detecting the mutant gene, both methods being directed towards achieving the aforementioned object. As a result, as shown in the conceptual view of Figure 1, the inventors have found that a mutant gene can be detected with high sensitivity by causing a fluorescence-labeled oligo having the sequence of a wild-type gene to hybridize with the wild-type gene, then carrying out a nucleic acid amplification reaction so that a mutant gene is preferentially amplified, and by then hybridizing the fluorescence-labeled oligo with the mutant gene, and thereafter measuring how much of light emission from a fluorescent dye changes before and after the hybridization; thus, the mutant gene can be detected with high sensitivity by using a single oligonucleotide that serves both as a clamp primer and as a fluorescence-labeled oligo. The present invention has been completed based on such findings.

Specifically, the present invention provides a method for specifically amplifying a specific gene sequence, which is characterized in that the above described oligonucleotide is hybridized with a wild-type gene in a gene group, and then, a nucleic acid amplification reaction is performed while suppressing the amplification of the wild-type gene so as to specifically amplify a mutant gene.

Moreover, the present invention provides a method for measuring a nucleic acid using a fluorescence-labeled oligo, which is characterized in that the oligonucleotide is such that when it hybridizes with a specifically amplified mutant gene, the fluorescent dye changes its light emission, and further characterized in that the above described oligonucleotide is hybridized with the mutant gene and a change in the light emission from the fluorescent dye before and after the hybridization is then measured.

Furthermore, the present invention provides: a fluorescence-labeled oligo which is characterized in that the above described fluorescent dye reduces its light emission when the fluorescence-labeled oligo is hybridized with a target nucleic acid, and that the oligonucleotide comprises nucleotides any one or more of which are composed of artificial nucleic acids; or a method for measuring a nucleic acid using the aforementioned fluorescence-labeled oligo.

The gist of the present invention is as follows.
(1) A method for measuring a nucleic acid for the purpose of specifically detecting a genotype as an examination target from subjects which are genes or specimens likely to have a plurality of genetic polymorphism, wherein
   the measurement method comprises hybridizing a fluorescent dye-labeled oligonucleotide (hereinafter referred to as a "fluorescence-labeled oligo") with a genotype other than the examination target to suppress the gene amplification of such genotype while at the same time, hybridizing the same fluorescence-labeled oligo as described above with an amplification product derived from the genotype of the examination target amplified in the same gene amplification step as described above, and specifically detecting the genotype of the examination target based on a change in the fluorescence intensity of the fluorescent dye before and after the hybridization.
(2) The method for measuring a nucleic acid according to the above (1), wherein
   the fluorescence-labeled oligo is labeled with a fluorescent dye at a terminal portion thereof,
   when the fluorescence-labeled oligo hybridizes with a target nucleic acid, the nucleotide sequence of the target nucleic acid has at least one nucleotide G (guanine) present in the range of 1 to 3 nucleotides counted from the terminal portion of the fluorescence-labeled oligo (wherein a target nucleic acid nucleotide forming a base pair with the fluorescence-labeled terminal portion is counted as 1), and
   the fluorescence-labeled oligo used has such a property that its fluorescence intensity is reduced by hybridization with the target nucleic acid.
(3) The method for measuring a nucleic acid according to the above (1) or (2), wherein
   the fluorescence-labeled oligo is labeled with a fluorescent dye at a terminal portion thereof,
   the nucleotide sequence of the fluorescence-labeled oligo is so designed that when it hybridizes with a target nucleic acid, the base pairs in the terminal portion form at least one base pair of G (guanine) and C (cytosine), and
   the fluorescence-labeled oligo used has such a property that its fluorescence intensity is reduced by hybridization with the target nucleic acid.
(4) The method for measuring a nucleic acid according to any one of the above (1) to (3), which uses a fluorescence-labeled oligo having such a nucleotide sequence that the number of mismatches upon hybridization with a nucleotide sequence comprising the genotype as the examination target is larger than the number of mismatches upon hybridization with a nucleotide sequence comprising a genotype other than the examination target.
(5) The method for measuring a nucleic acid according to any one of the above (1) to (4), which uses a fluorescence-labeled oligo in which part or all of the oligonucleotide is composed of artificial nucleic acids for increasing the dissociation temperature of a nucleic acid.
(6) The method for measuring a nucleic acid according to the above (5), wherein a fluorescence-labeled oligo, which uses at least one of artificial nucleic acids, namely, 2',4'-BNA^{coc}, 3'-Amino-2',4'-BNA, 2',4'-BNA^{NC} (in all of its appearances, BNA is an abbreviation for Bridged Nucleic Acid), PNA (Peptide Nucleic Acid), LNA (Locked Nucleic Acid), TNA (Threose nucleic acid), and GNA (Glycol nucleic acid), is used as an oligonucleotide for increasing the dissociation temperature of a nucleic acid.
(7) The method for measuring a nucleic acid according to any one of the above (1) to (6), wherein the gene amplification method is any one of PCR, LAMP, NASBA, ICAN, LCR, Rolling Cycle, SMAP, and PALSAR.
(8) The method for measuring a nucleic acid according to the above (1) to (7), wherein the gene amplification is carried out with a polymerase having 5' → 3' exonuclease activity.
(9) A fluorescence-labeled oligo that can be used in the method for measuring a nucleic acid according to any one of the above (1) to (8), wherein
   the fluorescence-labeled oligo is labeled with a fluorescent dye at a terminal portion thereof, and
   the nucleotide sequence of the fluorescence-labeled oligo is so designed that when it hybridizes with a target nucleic acid, the nucleotide sequence of the target nucleic acid has at least one nucleotide G (guanine) present in the range of 1 to 3 nucleotides counted from the terminal portion of the fluorescence-labeled oligo (wherein a target nucleic acid nucleotide forming a base pair with the fluorescence-labeled terminal portion is counted as 1).
(10) A fluorescence-labeled oligo that can be used in the method for measuring a nucleic acid according to any one of the above (1) to (8), wherein
   the fluorescence-labeled oligo is labeled with a fluorescent dye at a terminal portion thereof, and
   the nucleotide sequence of the fluorescence-labeled oligo is so designed that when it hybridizes with a target nucleic acid, the base pairs in the terminal portion form at least one base pair of G (guanine) and C (cytosine).

### Effects of Invention

According to the present invention, a mutant gene(s) in a gene group comprising wild-type genes can be detected with high sensitivity and/or high precision in a rapid and simple way. Specifically, according to the present invention, such a mutant gene(s) can be detected even if their relative content is approximately 0.1 %, and the measurement time is only approximately 1 hour. In addition, since only a single oligonucleotide is used, suitable oligonucleotide designing and condition characterization can easily be performed for a wide variety of gene mutations, and thus, the present provides a technique having extremely high versatility. Moreover, only one oligonucleotide is used other than a primer set, so the method of the present invention is reduced in cost. Furthermore, only the gene as an examination target can be specifically detected, so the method of the present invention is recognized to be a highly accurate technique that is adapted for application in clinical sites.

The present description includes part or all of the contents as disclosed in the specification and/or drawings of Japanese Patent Application No. 2015-70642, which is a priority document of the present application.

### Brief Description of Drawings

[Figure 1] Figure 1 shows a conceptual diagram of the present invention.
[Figure 2] Figure 2 shows the melting curves of the PCR reaction solutions of Example 1. The melting curves of solutions comprising target nucleic acids having mutation rates of 0%, 0.1%, 1%, and 100% are shown as A, B, C, and D, respectively. The melting curve of a solution comprising no target nucleic acid is shown as E.
[Figure 3] Figure 3 shows the negative first-order differential curves of the melting curves shown in Figure 2.
[Figure 4] Figure 4 shows the negative first-order differential curves of the melting curves for the PCR reaction solutions of Example 2. The negative first-order differential curves of the melting curves for solutions comprising target nucleic acids of samples 1, 2, and 3 are shown as A, B, and C, respectively. The negative first-order differential curve of the melting curve of a solution comprising no target nucleic acid is shown as D.
[Figure 5] Figure 5 shows the negative first-order differential curves of the melting curves for the PCR reaction solutions of Example 4 (using KOD plus). The designations 0%, 0.1 %, 0.5%, 1%, and 10% refer to mutation rates.
[Figure 6] Figure 6 shows the negative first-order differential curves of the melting curves for the PCR reaction solutions of Example 4 (using Takara Ex Taq HS). The designations 0%, 0.1%, 0.5%, 1%, and 10% refer to mutation rates.

### Description of Embodiments

Next, the present invention will be described in more detail in the following preferred embodiments. In the present invention, terms such as DNA, RNA, cDNA, mRNA, rRNA, NTPs, dNTPs, fluorescence-labeled oligo, hybridize, hybridization, intercalator, primer, annealing, elongation reaction, heat denaturation reaction, nucleic acid melting curve, PCR, RT-PCR, a PCR method using PNA, nucleic acid detection (gene detection) device, and SNP (single nucleotide polymorphism), have the same meanings as those that are currently in common used in molecular biology, genetic engineering, etc.

In the present invention, the term "wild-type gene" is used to mean a gene that has no mutations on its nucleotide sequence and comprises gene information that exhibits normal functions. The term "gene information" is used herein to include not only information encoding transcriptional regions such as rRNA or mRNA but also gene expression regulatory regions such as a promoter.

In the present invention, the term "mutant gene" is used to mean a gene having a mutation on its nucleotide sequence. The term "mutation" means a change on the nucleotide sequence of DNA or RNA, and it also includes insertion, deletion, translocation, and the like in genetics. However, a change in the gene function may not be generated by such mutation. The target of such mutation is not limited to a transcriptional region and it may also include a gene expression regulatory region such as a promoter.

In the present invention, the term "target nucleic acid" is used to mean a nucleic acid having the nucleotide sequences of the above described "wild-type gene" and "mutant gene," and it does not matter whether the target nucleic acid has been purified or not and how large its concentration is.

With regard to the fluorescence-labeled oligo that can be used in the present invention, a fluorescence-labeled oligo that is generally used in the measurement and/or detection of a nucleic acid can be conveniently used. Advantageous for use is such a fluorescence-labeled oligo that when it hybridizes with a target nucleic acid, the fluorescent dye with which the oligonucleotide is labeled changes its light emission. Specific examples of such a fluorescence-labeled oligo include Quenching Probe (Kurata et al., Nucleic Acids Research, Volume 29, Issue 6, e34), Universal quenching probe (Tani et al., Anal. Chem., 2009, 81 (14), pp 5678-5685), Molecular beacons (Tyagi et al., Nature Biotechnology 14, 303 - 308 (1996)), and Simple Probe (Lyon et al., J Mol Diagn. 2009 Mar; 11(2): 93-101).

Quenching Probe and Universal quenching probe are nucleic acid probes that utilize such a phenomenon that when a fluorescence-labeled oligo hybridizes with a target nucleic acid, the light emission from the fluorescent dye is quenched by a guanine nucleotide in the target nucleic acid. Molecular beacons is an oligonucleotide that is labeled with a fluorescent dye at its 5'-terminus and a quencher substance at its 3'-terminus and which assumes a loop structure that brings them closer to each other to cause quenching. This oligonucleotide serves as a nucleic acid probe which emits fluorescence when it hybridizes with a target nucleic acid. Simple Probe is a nucleic acid probe that utilizes such a phenomenon that when it hybridizes with a target nucleic acid, the labeling fluorescent dye emits fluorescence.

When Quenching Probe or Universal quenching probe is used in the present invention, fluorescent dyes that label the Quenching Probe or the Universal quenching probe for general use in nucleic acid measurement and/or detection can be conveniently used. Specifically, a fluorescent dye that is advantageously used is such that when it hybridizes with a target nucleic acid, is the fluorescent dye with which the oligonucleotide is labeled reduces its light emission. Examples of such a fluorescent dye include fluorescein or derivatives thereof {e.g., fluorescein isothiocyanate) (FITC) or a derivative thereof, etc., Alexa 488, Alexa532, cy3, cy5, EDANS (5-(2'-aminoethyl)amino-1-naphthalene sulfonic acid)}, rhodamine 6G (R6G) or derivatives thereof (e.g., tetramethylrhodamine (TMR), 5-(and 6)-carboxyrhodamine 6G (CR6G), tetramethylrhodamine isothiocyanate (TMRITC), x-rhodamine, Texas red, BODIPY FL(trademark name; manufactured by Thermo Fisher Scientific, U.S.A.), BODIPY FL/C3 (trademark name; manufactured by Thermo Fisher Scientific, U.S.A.), BODIPY FL/C6 (trademark name; manufactured by Thermo Fisher Scientific, U.S.A.), BODIPY 5-FAM (trademark name; manufactured by Thermo Fisher Scientific, U.S.A.), BODIPY TMR (trademark name; manufactured by Thermo Fisher Scientific, U.S.A.), or derivatives thereof (e.g., BODIPY TR (trademark name; manufactured by Thermo Fisher Scientific, U.S.A.), BODIPY R6G (trademark name; manufactured by Thermo Fisher Scientific, U.S.A.), BODIPY 564 (trademark name; manufactured by Thermo Fisher Scientific, U.S.A.), and BODIPY 581 (trademark name; manufactured by Thermo Fisher Scientific, U.S.A.). Among these substances, preferred examples of the fluorescent dye include FITC, EDANS, 6-joe, TMR, Alexa 488, Alexa 532, BODIPY FL/C3 (trademark name; manufactured by Thermo Fisher Scientific, U.S.A.), and BODIPY FL/C6 (trademark name; manufactured by Thermo Fisher Scientific, U.S.A.). More preferred examples of the fluorescent dye include FITC, TMR, 6-joe, BODIPY FL/C3 (trademark name; manufactured by Thermo Fisher Scientific, U.S.A.), BODIPY FL/C6 (trademark name; manufactured by Thermo Fisher Scientific), Pacific Blue (trademark name; manufactured by Thermo Fisher Scientific), ATTO 465 (trademark name; manufactured by ATTO-TEC), and ATTO 655 (trademark name; manufactured by ATTO-TEC).

When Quenching Probe or Universal quenching probe is used in the present invention, in order to ensure that the light emission from the fluorescent dye with which the oligonucleotide is labeled is changed efficiently, the nucleotide sequence of the target nucleic acid desirably has at least one nucleotide G (guanine) present in the range of 1 to 3 nucleotides counted from the terminal portion of the fluorescence-labeled oligo (wherein a target nucleic acid nucleotide forming a base pair with the fluorescence-labeled terminal portion is counted as 1), and the terminus may be more preferably designed to be G. Moreover, the nucleotide sequence of the fluorescence-labeled oligo which is labeled with the fluorescent dye in the terminal portion may be so designed that when it hybridizes with the target nucleic acid, it is the base pairs in the terminal portion of the fluorescence-labeled oligo form at least one base pair of G (guanine) and C (cytosine).

In the present invention, in order to suppress the amplification of only one of two genotypes, it is necessary to secure such a condition that in a temperature range over which elongation takes place (i.e., elongation generally takes place at around 72°C in PCR), the fluorescence-labeled oligo strongly binds to a target nucleic acid comprising a genotype whose amplification is to be suppressed whereas it does not bind strongly enough to a target nucleic acid comprising the other genotype (whose amplification is not to be suppressed) to suppress its amplification,. When there is a great difference in nucleotide sequence between a wild-type gene and a mutant gene, even if a fluorescence-labeled oligo composed of only naturally derived DNA is used, it is possible to ensure that the fluorescence-labeled oligo and a target nucleic acid comprising a genotype whose amplification is to be suppressed are dissociated at a temperature not lower than the temperature at which elongation takes place and also to ensure a sufficient difference between the temperature at which the fluorescence-labeled oligo is dissociated from a target nucleic acid comprising a genotype whose amplification is to be suppressed and the temperature at which the fluorescence-labeled oligo is dissociated from a target nucleic acid comprising a genotype whose amplification is not to be suppressed, and this makes it possible to secure the aforementioned condition (i.e., in a temperature range over which elongation takes place, the fluorescence-labeled oligo strongly binds to a target nucleic acid comprising a genotype whose amplification is to be suppressed whereas it does not bind strongly enough to a target nucleic acid comprising the other genotype whose amplification is not to be suppressed). On the other hand, when there is a very small difference in nucleotide sequence between a wild-type gene and a mutant gene (e.g., a case where only one nucleotide is mutated), it is difficult to create the same condition as that described above in a temperature range over which elongation takes place. In this case, by shortening the length of the fluorescence-labeled oligo, it is possible to ensure a sufficient difference between the temperature at which the fluorescence-labeled oligo binds to the wild-type gene and the temperature at which the fluorescence-labeled oligo binds to the mutant gene. However, as the result of shortening the length of the fluorescence-labeled oligo, the dissociation temperature of the fluorescence-labeled oligo decreases, making it difficult to ensure that in a temperature range over which elongation takes place (i.e., elongation generally takes place at around 72°C in PCR), the fluorescence-labeled oligo is allowed to strongly bind to a target nucleic acid comprising a genotype whose amplification is to be suppressed, whereby it becomes difficult to preferentially amplify one of the two genotypes. In such a case, an artificial nucleic acid characterized in that it increases the dissociation temperature, such as 2',4'-BNA^{coc}, 3'-Amino-2',4'-BNA, 2',4'-BNA^{NC} (in all of its appearances, BNA is an abbreviation for Bridged Nucleic Acid), PNA (Peptide Nucleic Acid), LNA (Locked Nucleic Acid), TNA (Threose nucleic acid), or GNA (Glycol nucleic acid), can advantageously be used as a nucleotide that constitutes the fluorescence-labeled oligo. By using such artificial nucleic acid, even under circumstances where the difference in nucleotide sequence between a wild-type gene and a mutant gene is so small that the length of the fluorescence-labeled oligo has to be shortened, it becomes possible to easily realize such a condition that in a temperature range over which elongation takes place, the fluorescence-labeled oligo firmly binds to a target nucleic acid comprising a genotype whose amplification is to be suppressed whereas it does not strongly bind to a genotype whose amplification is not to be suppressed. The site into which the artificial nucleic acid is to be inserted may be a chimeric oligonucleotide which is a mixture of the artificial nucleic acid and natural DNA, or it may be entirely composed of artificial nucleic acids, and thus, the site is not particularly limited. In a desired embodiment, an artificial nucleic acid(s) may be inserted into a nucleotide sequence portion that is different between a wild-type gene and a mutant gene.

As described above, it is important that nucleotides that constitute the fluorescence-labeled oligo should be optimized with natural nucleic acids, artificial nucleic acids, a combination thereof, or the like, depending on a difference in nucleotide sequence between a wild-type gene and a mutant gene. The types of such nucleic acids are not particularly limited in the present invention.

In the present invention, the term "clamp primer" is used to mean such an oligonucleotide that the temperature at which it is dissociated from a target nucleic acid comprising a genotype whose amplification is to be suppressed is higher than the temperature at which it is dissociated from a target nucleic acid comprising a genotype whose amplification is not to be suppressed and this term refers to the same oligonucleotide as the fluorescence-labeled oligo. The present embodiment is characterized in that the number of hydrogen bonds between the clamp primer and a target nucleic acid comprising a genotype whose amplification is to be suppressed (the number of base pairs) is larger than the number of hydrogen bonds between the clamp primer and a target nucleic acid comprising a genotype whose amplification is not to be suppressed (the number of base pairs). It is desirable that the length of the clamp primer consists of 10 to 25 nucleotides, and that the Tm value is 70°C to 100°C. The concentration of the clamp primer in an amplification reaction solution is desirably 10 to 500 nM, and more preferably about 20 to 200 nM. A difference between the Tm of a complex consisting of the clamp primer and a target nucleic acid comprising a genotype whose amplification is not to be suppressed and the Tm of a complex consisting of the clamp primer and a target nucleic acid comprising a genotype whose amplification is to be suppressed is suitably about 5°C to 25°C, preferably 10°C to 25°C, and more preferably 10°C to 20°C. The Tm of a complex consisting of the clamp primer and a target nucleic acid comprising a genotype whose amplification is not to be suppressed can be set at about 40°C to 80°C, and it is suitably 50°C to 75°C. The Tm of a complex consisting of the clamp primer and a target nucleic acid comprising a genotype whose amplification is to be suppressed can be set at about 60°C to 90°C, and it is suitably 70°C to 85°C.

In the present invention, the term "fluorescence-labeled oligo" is used to mean an oligonucleotide that hybridizes with a target nucleic acid, and this term refers to the same oligonucleotide as the clamp primer. In the present embodiment, such fluorescence-labeled oligo has a sequence that is so designed that the number of hydrogen bonds between the fluorescence-labeled oligo and a target nucleic acid comprising a genotype whose amplification is to be suppressed (the number of base pairs) is larger than the number of hydrogen bonds between the fluorescence-labeled oligo and a target nucleic acid comprising a genotype whose amplification is not to be suppressed (the number of base pairs). Briefly, there is used a fluorescence-labeled oligo having such a nucleotide sequence that the number of mismatches upon hybridization with a nucleotide sequence comprising the genotype as the examination target is larger than the number of mismatches upon hybridization with a nucleotide sequence comprising a genotype other than the examination target. Moreover, the present fluorescence-labeled oligo is characterized in that a change in the light emission from the labeling fluorescent dye is measured before and after its hybridization with the target nucleic acid, to thereby detect the target nucleic acid. Furthermore, the fluorescence-labeled oligo is such that there is a difference between the dissociation temperature for the binding of the oligonucleotide to a target nucleic acid comprising a genotype whose amplification is to be suppressed and the dissociation temperature for the binding of the oligonucleotide to a target nucleic acid comprising a genotype whose amplification is not to be suppressed, so the present fluorescence-labeled oligo has such a function that by measuring the difference between the two dissociation temperatures, a target nucleic acid comprising a genotype whose amplification is to be suppressed can be distinguished from a target nucleic acid comprising a genotype whose amplification is not to be suppressed. An advantageous method for measuring a difference between the two dissociation temperatures may be exemplified by a melting curve analysis capable of recognizing a dissociation temperature by measuring a change in the fluorescence of the fluorescence-labeled oligo while changing the temperature.

In the present invention, the term "nucleic acid amplification method" is used to mean a method for amplifying a detection region comprising a target sequence using an amplification primer, and the way of performing this method is not particularly limited. For example, the nucleic acid amplification method may be any one of PCR, LAMP, NASBA, ICAN, LCR, Rolling Cycle, SMAP, and PALSAR.

The above described "amplification primer" is used to mean a nucleic acid used to amplify a detection region in the nucleic acid amplification method. With regard to the concentration of the amplification primer, an optimal concentration may be determined in a range over which amplification takes place. Generally speaking, a concentration of 100 nM to 1.5 µM is set in many cases. Moreover, the concentration of an amplification primer that hybridizes with the same side as the fluorescence-labeled oligo functioning as a clamp primer is desirably higher than the concentration of an amplification primer on the opposite side, and in many cases, the concentration of the amplification primer that hybridizes with the same side as the fluorescence-labeled oligo functioning as a clamp primer is advantageously set at a level that is about 1.5 to 10 times higher than the concentration on the opposite side.

The number of nucleotides that comprise an amplification primer is desirably 10 to 40 nucleotides, and more preferably about 15 to 35 nucleotides. The sequence of such an amplification primer is not particularly limited as long as it is capable of amplifying a detection region comprising a target sequence according to a nucleic acid amplification method. The Tm value is suitably about 45°C to 80°C, preferably 50°C to 70°C, and more preferably 55°C to 65°C.

As for the temperature in the annealing step of an amplification cycle, it is the temperature at which an amplification primer is sufficiently hybridized and it is set in the range of -20°C to +10°C relative to the Tm of a complex consisting of a nucleic acid sequence whose amplification is not to be suppressed and a clamp primer. More preferably, this temperature is desirably set in the range of -20°C to 0°C, and even more preferably -10°C to 0°C, relative to the aforementioned Tm value. The annealing temperature in the amplification cycle can be set in the range of 40°C to 75°C.

Enzymes applicable for gene amplification include an enzyme having 5'-3' exonuclease activity and an enzyme not having 5'-3' exonuclease activity. If either of these enzymes can be utilized in the present invention, selection can be made from a wide range of enzymes. Consequently, the possibility of improving performance as in sensitivity and precision, or the possibility of reducing production costs and the like is increased, and eventually, the possibility of making more competitive products and/or services by utilizing the present invention is increased.

On the other hand, if an enzyme having 5'-3' exonuclease activity is used in the present invention, there is a likelihood that the clamp primer will be decomposed, the amplification of a non-target nucleic acid will not be suppressed, and highly sensitive detection of a target nucleic acid cannot be achieved.

Hence, a check was made to evaluate the applicability of the present invention in two cases, one of using an enzyme having 5'-3' exonuclease activity and another of using an enzyme not having 5'-3' exonuclease activity. As it turned out, both enzymes could successfully be used and a target nucleic acid could be detected with high sensitivity (Example 4 to be described later). The present invention also includes technical contents based on the above described findings.

The measurement principle of the present invention is as described above, and it can be applied to various types of nucleic acid measurement methods. Hereafter, some examples are given.

Quenching Probe functioning both as a clamp primer complementary to the target sequence of a wild-type gene and as a fluorescence-labeled oligo, an amplification primer, and DNA comprising a target gene group are mixed with a reaction solution used for gene amplification. As a result, the Quenching Probe preferentially hybridizes with the wild-type gene whereas the amplification primer hybridizes with the wild-type and mutant genes at the same efficiency.

Subsequently, according to a gene amplification reaction, elongation of the amplification primer is carried out. As a result of the hybridization with the Quenching Probe, elongation is suppressed in the detection region of the wild-type gene whereas a detection region comprising the target sequence of the mutant gene is preferentially amplified.

After completion of the elongation reaction, the Quenching Probe and the amplification primer are hybridized again with gene sequences that are respectively complementary to them. In the case of a PCR reaction, heat denaturation has to be carried out at around 95°C; Other amplification methods such as LAMP and ICAN allow reaction to be carried out at a constant temperature. By repeating the cycles of the gene amplification reaction, the detection region of the mutant gene can be selectively amplified. The number of cycles is desirably about 30 to 55.

The detection region of the mutant gene as amplified by the above described gene amplification method is detected. The detection is carried out using Quenching Probe labeled with a fluorescent dye at the 3'- or 5'-terminus. The Quenching Probe hybridizes with the mutant gene which has been preferentially amplified, although there is one nucleotide mismatch. Subsequently, the temperature dependence of the fluorescence intensity of the fluorescent dye is measured. Specifically, as the temperature of the solution is changed from low to high, the fluorescence intensity of the fluorescent dye is measured at each temperature.

A plot of the fluorescence intensity of the fluorescent dye against temperature is referred to as a melting curve. By differentiating the melting curve with respect to temperature, two Tm values, one for a complex consisting of the Quenching Probe and a mutant gene and another for a complex consisting of the Quenching Probe and a normal gene, can be obtained as temperatures indicating extremums. Such a melting curve analysis can be carried out easily by using a commercially available program well known to persons skilled in the art.

The light emission from the fluorescent dye in a reaction solution comprising the above described Quenching Probe is suppressed at low temperature by the quenching phenomenon due to guanine in the target sequence located close to the fluorescent dye. If the temperature is increased to around the Tm value of a complex consisting of the Quenching Probe and a mutant gene, the Quenching Probe is dissociated, the quenching degree is attenuated, and the fluorescence intensity is increased. Accordingly, a mutant gene can be easily detected by carrying out the melting curve analysis.

Moreover, if a nucleotide having a guanine nucleotide which exhibits a quenching action on a fluorescent substance with which the Quenching Probe is labeled is mutated in the nucleotide sequence of a target nucleic acid, a decrease in the fluorescence does not occur at any temperature, so the mutation can be identified by the melting curve analysis.

Hereinafter, the present invention will be more specifically described in the following examples. However, these examples are provided only for illustrating the present invention, and are not intended to limit the scope of the present invention.

### Examples

### [Example 1] High-sensitivity detection of JAK2 gene mutation (model system that targets PCR products)

Templates for a PCR reaction were each prepared to give a total of 10000 copies/µl, such that the ratio between wild-type genes and mutant genes in a partial PCR product (363 bp) of a human JAK2 gene sequence would be 0:100, 90:10, 99:1, 99.5:0.5, 99.9:0.1, and 100:0. Each reaction solution comprised 1 µl of template DNA (10000 copies), KOD plus DNA polymerase (Toyobo Co., Ltd.) used as DNA polymerase, 4 dNTPs (0.2 mM each), a forward primer (SEQ ID NO: 1, final concentration: 1.0 µM), a reverse primer (SEQ ID NO: 2, final concentration: 0.2 µM), a magnesium sulfate solution (final concentration: 1 mM), a predetermined amount of KOD plus polymerase, and Quenching Probe (SEQ ID NO: 3, final concentration: 0.05 µM) labeled with carboxyrhodamine 6G (CR6G) at the 3'-terminus. It is to be noted that the Quenching Probe functions both as a clamp primer and as a fluorescence-labeled oligo for detection of a target nucleic acid. Each PCR reaction solution was prepared by adding sterilized water to make 15 µl.

In the following sequences, nucleotides prefixed with the symbol + are each composed of 2',4'-BNA^{NC}, and other nucleotides each composed of DNA.
SEQ ID NO: 1: ATCTATAGTCATGCTGAAAGTAGGAGAAA (29 nucleotides)
SEQ ID NO: 2: CTGAATAGTCCTACAGTGTTTTCAGTTTCA (30 nucleotides)
SEQ ID NO: 3: +C+AC+A+G+A+C+A+C+AT+A+C+T+C+C (16 nucleotides) - CR6G

The above described reaction solution was subjected to the following PCR reaction, using a real-time PCR apparatus (Rotor-Gene (Qiagen)).
(1) Heat denaturation step: 95°C, 300 seconds
(2) Heat denaturation step: 95°C, 10 seconds
(3) Annealing step: 60°C, 30 seconds
(4) Elongation step: 68°C, 20 seconds
(5) Temperature-increasing step: 50°C to 99°C

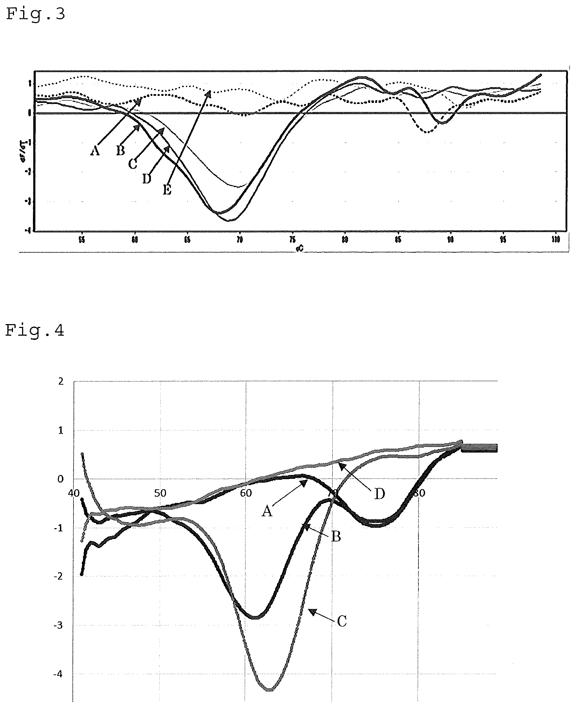

After completion of the heat denaturation step (1), the steps (2) to (4) were repeatedly carried out through 50 cycles. In the temperature-increasing step (5), fluorescence intensity was measured to construct melting curves as shown in Figure. In the figure, the melting curves for solutions comprising target nucleic acids at mutation rates of 0%, 0.1%, 1%, and 100% are indicated by A, B, C and D, respectively, and the melting curve for a solution comprising no target nucleic acids is indicated by E. In addition, the negative first-order differential curves of these melting curves are shown in Figure 3.

As a result of experiments, by performing a melting curve analysis using the Quenching Probe of the present invention, minimum peaks were obtained in the negative first-order differential curves of the melting curves B, C and D in Figure 3 at the melting temperature of a complex of the above described Quenching Probe and the target nucleic acid, whereas no such peak was detected in the negative first-order differential curves of the melting curves A and E.

From these results, it became clear that by using the above described Quenching Probe, a mutation in the JAK2 gene group could be clearly detected when its content was at least 0.1%.

### [Example 2] High-sensitivity detection of JAK2 gene mutation (actual samples)

Genomic DNA extracted from the blood of a patient with myeloproliferative neoplasm was used as template DNA in PCR. It is to be noted that such DNA extraction was carried out using QIAamp DNA Mini Kit (Qiagen). The reaction solutions were each prepared from2.4 µl of a mixed solution comprising PPD mix (Toyobo Co., Ltd.), a forward primer (SEQ ID NO: 1, final concentration: 1.2 µM) and a reverse primer (SEQ ID NO: 4, final concentration: 0.2 µM) dissolved in the PPD mix, and Quenching Probe (SEQ ID NO: 5, final concentration: 0.12 µM) labeled with carboxyrhodamine 6G (CR6G) at the 3'-terminus, 3.6 µl of KOD mix (Toyobo Co., Ltd.), and 6 µl of a solution containing 30 ng of genomic DNA and sterilized water to give a total of 12 µl. It is to be noted that as in [Example 1], the Quenching Probe functions both as a clamp primer and as a fluorescence-labeled oligo for detection of a target nucleic acid.

In the following sequences, nucleotides prefixed with the symbol + are each composed of 2',4'-BNA^{NC}, and other nucleotides each composed by DNA.
SEQ ID NO: 4: CACCTAGCTGTGATCCTGAA (20 nucleotides)
SEQ ID NO: 5: +C+AC+AG+A+C+AC+AT+AC+TC+C (16 nucleotides) - CR6G

The above described reaction solution was subjected to the following PCR reaction, using a gene analysis apparatus (GENECUBE, manufactured by Toyobo Co., Ltd.).
(1) Heat denaturation step: 95°C, 30 seconds
(2) Heat denaturation step: 95°C, 2 seconds
(3) Annealing step: 60°C, 3 seconds
(4) Elongation step: 68°C, 5 seconds
(5) Temperature-increasing step: 40°C to 99°C

After completion of the heat denaturation step (1), the steps (2) to (4) were repeatedly carried out through 55 cycles. In the temperature-increasing step (5), fluorescence intensity was measured to construct melting curves. Negative first-order differential curves of the melting curve are shown in Figure 4. In the figure, negative first-order differential curves of the melting curves for solutions comprising the target nucleic acids of samples 1, 2, and 3 are indicated by A, B, and C, and a negative first-order differential curve of the melting curve for a solution comprising no target nucleic acids is indicated by D.

As a result of experiments, by performing a melting curve analysis using the Quenching Probe of the present invention, a minimum peak was obtained in the negative first-order differential curve of the melting curve A in Figure 4 at the melting temperature of a complex consisting of the above described Quenching Probe and a normal gene. On the other hand, minimum peaks were obtained in the negative first-order differential curve of the melting curve B in Figure 4 at the melting temperatures of two complexes, one consisting of the Quenching Probe and a normal gene, and another consisting of the Quenching Probe and a mutant gene; in addition, a minimum peak was obtained in the negative first-order differential curve of the melting curve C in Figure 4 at the melting temperature of a complex consisting of the Quenching Probe and a mutant gene. Moreover, no peak was detected in the negative first-order differential curve of the melting curve D in Figure 4.

The results of evaluation by the present technique and with the use of a next-generation sequencer, as well as mutation rates estimated by a semi-quantitative analysis are shown in Table 1. Two samples (A and B) estimated to have mutation rates of no more than 0.1% gave the same results of evaluation as those obtained with the use of the next-generation sequencer. Consequently, it might be concluded that by using the above described Quenching Probe, a mutation can be detected from genomic DNA if its content n JAK2 genes is at least 0.1%.

**[Table 1]**

| sample | The present technique | Next-generation sequencer | Semi-quantitative results |
|---|---|---|---|
| A | × | × | 0-0.1% |
| B | ○ | ○ | 0-0.1% |
| C | ○ | Not performed | 1-10% |

### [Example 3] Studies using different artificial nucleic acids (actual samples)

For the purpose of confirming the effectiveness of the present method in the case of using different artificial nucleic acids, an experiment was carried out using Quenching Probe composed of 2',4'-BNA^{NC}, PNA, LNA, or natural DNA. Specifically, with regard to Quenching Probes prepared in the present example, a total of four Quenching Probes were synthesized by using 2',4'-BNA^{NC}, PNA, LNA, or natural DNA, as nucleotides prefixed with the symbol + in SEQ ID NO: 3 in [Example 1]. The Quenching Probes were designed to be identical to one another. It is to be noted that as in [Example 1] and [Example 2], the present Quenching Probe functions both as a clamp primer and as a fluorescence-labeled oligo for detection of a target nucleic acid.

The results of the experiment are shown in Table 2.

The ratio of mutant genes that could be detected in the case of using the fluorescence-labeled oligo decreased in the following order: Quenching Probe composed of 2',4'-BNA^{NC} and natural nucleic acid (DNA) > Quenching Probe composed of LNA and natural nucleic acid > Quenching Probe composed of PNA and natural nucleic acid > Quenching Probe entirely composed of natural nucleic acid (DNA). From these results, it was suggested that the use of an artificial nucleic acid is preferable if there is only a small difference in sequence, as in the case of the target nucleic acid of the present example (specifically, in the case of one nucleotide mutation), it has been. It was also suggested that the detection limit of a mutant gene varies with the species of artificial nucleic acids, and that 2',4'-BNA^{NC} has the highest functionality of all the artificial nucleic acids studied.

**[Table 2]**

| Nucleotide species in fluorescence-labeled oligo | Ratio of mutant genes at detection limit |
|---|---|
| Quenching Probe composed of 2',4'-BNA^{NC} and natural nucleic acid (DNA) | 99.9 : 0.1 (0.1%) |
| Quenching Probe composed of LNA and natural nucleic acid (DNA) | 99.5 : 0.5 (0.5%) |
| Quenching Probe composed of PNA and natural nucleic acid (DNA) | 99 : 1 (1%) |
| Quenching Probe entirely composed of natural nucleic acid (DNA) | 90 : 10 (10%) |

### [Example 4] Studies regarding sensitivity that depends on type of polymerase

Templates for a PCR reaction were each prepared to give a total of 10000 copies/µl, such that the ratio between wild-type genes and mutant genes in a partial PCR product (151 1] bp) of a human MPL gene sequence would be 90:10, 99:1, 99.5:0.5, 99.9:0.1, and 100:0. Each reaction solution comprised 1 µl of template DNA (10000 copies), a predetermined amount of KOD plus DNA polymerase (Toyobo Co., Ltd.) or Takara Ex Taq HS (Takara Bio, Inc.) used as DNA polymerase, 4 dNTPs (0.2 mM each), a forward primer (SEQ ID NO: 6, final concentration: 1.0 µM), a reverse primer (SEQ ID NO: 7, final concentration: 0.1 µM), a magnesium sulfate solution (final concentration: 1 mM), and Quenching Probe (SEQ ID NO: 8, final concentration: 0.05 µM) labeled with carboxyrhodamine 6G (CR6G) at the 3'-terminus. Herein, KOD plus DNA polymerase is an example of an enzyme that does not have 5'-3' exonuclease activity but has 3'-5' exonuclease activity whereas Takara Ex Taq HS is an example of an enzyme that has 5'-3' exonuclease activity but does not have 3'-5' exonuclease activity. It is to be noted that the Quenching Probe functions both as a clamp primer and as a fluorescence-labeled oligo for detection of a target nucleic acid. Each PCR reaction solution was prepared by adding sterilized water to make 15 µl.

In the following sequences, nucleotides prefixed with the symbol + are each composed of 2',4'-BNA^{NC}, and other nucleotides each composed of DNA.
SEQ ID NO: 6: TGACCGCTCTGCATCTAGTGC (21 nucleotides)
SEQ ID NO: 7: GGTCACAGAGCGAACCAAGA (20 nucleotides)
SEQ ID NO: 8: +AC+TGC+CA+CC+TCA+GC+AG+C (17 nucleotides) - CR6G

The above described reaction solution was subjected to the following PCR reaction, using a real-time PCR apparatus (Rotor-Gene (Qiagen)).
(1) Heat denaturation step: 95°C, 300 seconds
(2) Heat denaturation step: 95°C, 10 seconds
(3) Annealing step: 58°C, 30 seconds
(4) Elongation step: 68°C, 20 seconds
(5) Temperature-increasing step: 50°C to 99°C

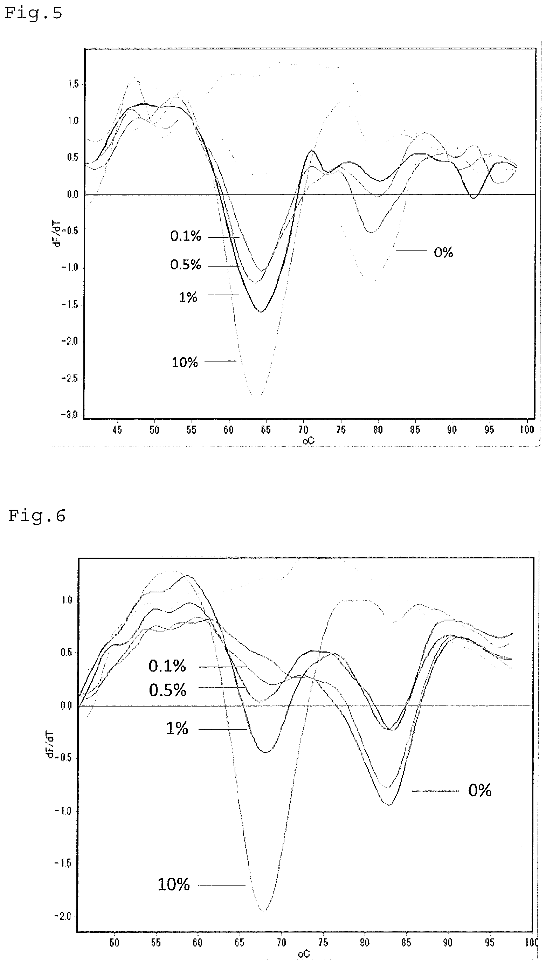

After completion of the heat denaturation step (1), the steps (2) to (4) were repeatedly carried out through 50 cycles. In the temperature-increasing step (5), fluorescence intensity was measured and the negative first-order differential curves of the obtained melting curves are shown in Figures 5 and 6 (wherein KOD plus and Takara Ex Taq HS were respectively used).

As a result of the experiment, whichever type of DNA polymerase was used, a mutant gene could be detected from a sample having a mutation rate of 0.1%and no substantial in sensitivity was observed. Consequently, it was demonstrated that the present invention can be used regardless of the type of DNA polymerase, in particular, the presence or absence of 5'-3' exonuclease activity or 3'-5' exonuclease activity.

All publications, patents and patent applications cited in the present description are incorporated herein by reference in their entirety.

### Industrial Applicability

The present invention can be utilized in detection of a gene mutation.

### Sequence Listing Free Text

<SEQ ID NO: 1>
   SEQ ID NO: 1 shows the nucleotide sequence of the forward primer used in Example 1.
<SEQ ID NO: 2>
   SEQ ID NO: 2 shows the nucleotide sequence of the reverse primer used in Example 1.
<SEQ ID NO: 3>
   SEQ ID NO: 3 shows the nucleotide sequence of the Quenching Probe used in Example 1.
<SEQ ID NO: 4>
   SEQ ID NO: 4 shows the nucleotide sequence of the reverse primer used in Example 2.
<SEQ ID NO: 5>
   SEQ ID NO: 5 shows the nucleotide sequence of the Quenching Probe used in Example 2.
<SEQ ID NO: 6>
   SEQ ID NO: 6 shows the nucleotide sequence of the forward primer used in Example 4.
<SEQ ID NO: 7>
   SEQ ID NO: 7 shows the nucleotide sequence of the reverse primer used in Example 4.
<SEQ ID NO: 8>
   SEQ ID NO: 8 shows the nucleotide sequence of the Quenching Probe used in Example 4.

## Claims

1. A method for measuring a nucleic acid for the purpose of specifically detecting a genotype as an examination target from subjects which are genes or specimens likely to have a plurality of genetic polymorphisms, wherein
the measurement method comprises hybridizing a fluorescent dye-labeled oligonucleotide (hereinafter referred to as a "fluorescence-labeled oligo") with a genotype other than the examination target to suppress the gene amplification of such genotype while at the same time, hybridizing the same fluorescence-labeled oligo as described above with an amplification product derived from the genotype of the examination target amplified in the same gene amplification step as described above and specifically detecting the genotype of the examination target based on a change in the fluorescence intensity of the fluorescent dye before and after the hybridization.

2. The method for measuring a nucleic acid according to claim 1, wherein
the fluorescence-labeled oligo is labeled with a fluorescent dye at a terminal portion thereof,
when the fluorescence-labeled oligo hybridizes with a target nucleic acid, the nucleotide sequence of the target nucleic acid has at least one nucleotide G (guanine) present in the range of 1 to 3 nucleotides counted from the terminal portion of the fluorescence-labeled oligo (wherein a target nucleic acid nucleotide forming a base pair with the fluorescence-labeled terminal portion is counted as 1), and
the fluorescence-labeled oligo used has such a property that its fluorescence intensity is reduced by hybridization with the target nucleic acid.

3. The method for measuring a nucleic acid according to claim 1 or 2, wherein
the fluorescence-labeled oligo is labeled with a fluorescent dye at a terminal portion thereof
the nucleotide sequence of the fluorescence-labeled oligo is so designed that when it hybridizes with a target nucleic acid, the base pairs in the terminal portion form at least one base pair of G (guanine) and C (cytosine), and
the fluorescence-labeled oligo used has such a property that its fluorescence intensity is reduced by hybridization with the target nucleic acid.

4. The method for measuring a nucleic acid according to any one of claims 1 to 3, which uses a fluorescence-labeled oligo having such a nucleotide sequence that the number of mismatches upon hybridization with a nucleotide sequence comprising the genotype as the examination target is larger than the number of mismatches upon hybridization with a nucleotide sequence comprising a genotype other than the examination target.

5. The method for measuring a nucleic acid according to any one of claims 1 to 4, which uses a fluorescence-labeled oligo in which part or all of the oligonucleotide is composed of artificial nucleic acids for increasing the dissociation temperature of a nucleic acid.

6. The method for measuring a nucleic acid according to claim 5, wherein a fluorescence-labeled oligo, which uses at least one of artificial nucleic acids, namely, 2',4'-BNA^{coc}, 3'-Amino-2',4'-BNA, 2',4'-BNA^{NC} (in all of its appearances, BNA is an abbreviation for Bridged Nucleic Acid), PNA (Peptide Nucleic Acid), LNA (Locked Nucleic Acid), TNA (Threose nucleic acid), and GNA (Glycol nucleic acid), is used as an oligonucleotide for increasing the dissociation temperature of a nucleic acid.

7. The method for measuring a nucleic acid according to any one of claims 1 to 6, wherein the gene amplification method is any one of PCR, LAMP, NASBA, ICAN, LCR, Rolling Cycle, SMAP, and PALSAR.

8. The method for measuring a nucleic acid according to claims 1 to 7, wherein the gene amplification is carried out with a polymerase having 5' → 3' exonuclease activity.

9. A fluorescence-labeled oligo that can be used in the method for measuring a nucleic acid according to any one of claims 1 to 8, wherein
the fluorescence-labeled oligo is labeled with a fluorescent dye at a terminal portion thereof, and
the nucleotide sequence of the fluorescence-labeled oligo is so designed that when it hybridizes with a target nucleic acid, the nucleotide sequence of the target nucleic acid has at least one nucleotide G (guanine) present in the range of 1 to 3 nucleotides counted from the terminal portion of the fluorescence-labeled oligo (wherein a target nucleic acid nucleotide forming a base pair with the fluorescence-labeled terminal portion is counted as 1).

10. A fluorescence-labeled oligo that can be used in the method for measuring a nucleic acid according to any one of claims 1 to 8, wherein
the fluorescence-labeled oligo is labeled with a fluorescent dye at a terminal portion thereof, and
the nucleotide sequence of the fluorescence-labeled oligo is so designed that when it hybridizes with a target nucleic acid, the base pairs in the terminal portion form at least one base pair of G (guanine) and C (cytosine).
